Europäisches Patentamt

**European Patent Office**

Office européen des brevets

(11) Publication number: **0 168 967**
A1

# (12) EUROPEAN PATENT APPLICATION

(21) Application number: **85304254.7**

(22) Date of filing: **14.06.85**

(51) Int. Cl.⁴: **A 61 F 2/48, A 61 F 5/44**

(30) Priority: **15.06.84 US 620846**

(43) Date of publication of application: **22.01.86**
Bulletin 86/4

(84) Designated Contracting States: **AT BE CH DE FR GB IT
LI LU NL SE**

(71) Applicant: **WATERS INSTRUMENTS, INC., P.O.
Box 6117, Rochester Minnesota 55901 (US)**

(72) Inventor: **Burton, Thomas A., 822 Sierra Lane, N.E.,
Rochester Minnesota 55901 (US)**

(74) Representative: **Carpmael, John William Maurice et al,
CARPMAELS & RANSFORD 43 Bloomsbury Square,
London, WC1A 2RA (GB)**

(54) **Ostomy prosthesis.**

(57) An ostomy prosthesis for controlling bowel continence is
provided with a drainage tube having a generally rigid inner por-
tion (14) and a limp, readily collapsed outer portion (16) which
extends from the stoma (S) of a patient. Securing means, such
as an inflatable balloon (30), is carried by the inner end portion
of the drainage tube deep to the facia (F). For securely holding
and sealing the outer end portion of drainage tubing used in an
ostomy prosthesis, a plate (40) is provided having a central
aperture (42) through which the outer end portion of drainage
tubing (16) may protrude, the plate including folding means for
folding the tubing into a Z-shaped configuration and further in-
cluding cover means (60) carried by the plate for covering and
pressing inwardly upon the folding tubing sections to secure
the same in place.

ACTORUM AG

## OSTOMY PROSTHESIS

The invention is in the field of medical prosthetic devices, and particularly devices intended for use with ileostomy or colostomy patients.

In now routine surgical operations referred to as ileostomies or colostomies, a portion of the bowel is removed and the end of the remaining bowel is brought out through the patient's abdomen. The end of the remaining bowel either terminates at or very close to the surface of the patient's skin, or may protrude slightly from the outer skin surface. The bowel end portion passes through the abdominal facia, muscle, fat and skin layers, and the end portion of the bowel is referred to as the "stoma".

Patients who have had such surgical operations often are provided with externally worn containers such as plastic bags, the bags having openings that are adhesively sealed to the skin about the stoma. A short tube may be anchored at one end within the stoma with its outer end extending outwardly for attachment to a receptacle; tubes of this type are shown in U.S. Patents 3,828,782 and 4,119,100.

Ileostomy and colostomy patients may encounter a variety of physical and psychological problems.

From the physical standpoint, such patients often must
carefully control their diets, and continuously must
contend with bags and other items of equipment. Bowel
contents which come into contact with the skin of a
patient surrounding the stoma can irritate and infect
the skin surface; such infections may become particularly threatening because they are so difficult to
heal inasmuch as the infected area continuously becomes reinfected through contact with bowel contents.

U.S. Patent 4,381,765 described an ileostomy
valve having a drainage tube insertable in the stoma
of a patient. The drainage tube was illustrated as
having a balloon-like structure at its inner end which
was carried within the bowel deep to the facia and
which prevented the drainage tube from being pulled
forwardly and outwardly of the stoma. The outer end
portion of the drainage tube was depicted as including
a valve body having an orifice through which the
drainage tube extended. In use, the valve body would
be slid rearwardly along the drainage tube toward the
patient's abdomen, and would then be held in place by
appropriate bending.

Although the ileostomy valve described in U.S.
Patent 4,381,765 has given excellent results in actual
usage, there is a tendency for the rather thick-walled
drainage tube, which is under continuous tension, to
creep and thus to withdraw gradually from the valve
body. Also, the outer, sealed end portion of the
drainage tube was continuously exposed and could be
considered, at least by some, to be unsightly.

The present invention in one embodiment provides an ostomy prosthesis that comprises a drainage
tube having inner and outer end portions, the inner end portion being receivable in the stoma of a patient deep to the facia and having walls of sufficient rigidity as to maintain a generally circular, open cross-section, the outer end portion being connected to and communicating with the inner end portion and having limp, readily collapsible walls which are collapsed inwardly by the stoma of a patient. Reversible securing means, desirably a toroidal balloon structure, is carried by the inner end portion of the drainage tube to reversibly secure the same to and within the stoma deep to the facia, and sealing means are carried by the limp, collapsible outer end portion of the drainage tube to releasably seal the latter against escape of bowel contents. In a preferred embodiment, the drainage tube includes a generally rigid section of tubing defining the inner end portion and a limp, easily collapsible section of tubing defining the outer end portion, the limp tubing being drawn over the more rigid tubing and being concentrically sealed to the more rigid tubing at at least two points spaced along the length of the more rigid tubing to define inflatable balloon means, the prosthesis including means for inflating the balloon means.

In another embodiment, the invention relates to means for releasably sealing the outer end portion of a drainage tube such as the tube described above. The sealing means comprises plate means carried by the tube exterior of the patient for releasably shaping the limp, readily collapsible outer end portion in a bent, collapsed, generally Z-shaped orientation to seal that portion against the escape of bowel contents. The sealing means includes cover means overlying the bent, collapsed outer end portion of the drainage tube and closable upon the plate means to maintain the drainage tubing in the bent, sealed orientation as thus described.

Figure 1 is a cross-sectional view of a drainage tube of the invention, shown broken away and in partial cross-section;

Figure 2 is a cross-sectional view of a prosthesis of the invention shown partially installed in the stoma of a patient;

Figure 3 is a broken-away view taken along line 3-3 of Figure 2;

Figure 4 is a front view of a portion of the prosthesis shown in Figure 2;

Figure 5 is a cross-sectional view taken along line 5-5 of Figure 4;

Figure 6 is a plan view of a modified device of the type shown in Figure 4; and

Figure 7 is a cross-sectional view taken along line 7-7 of Figure 6.

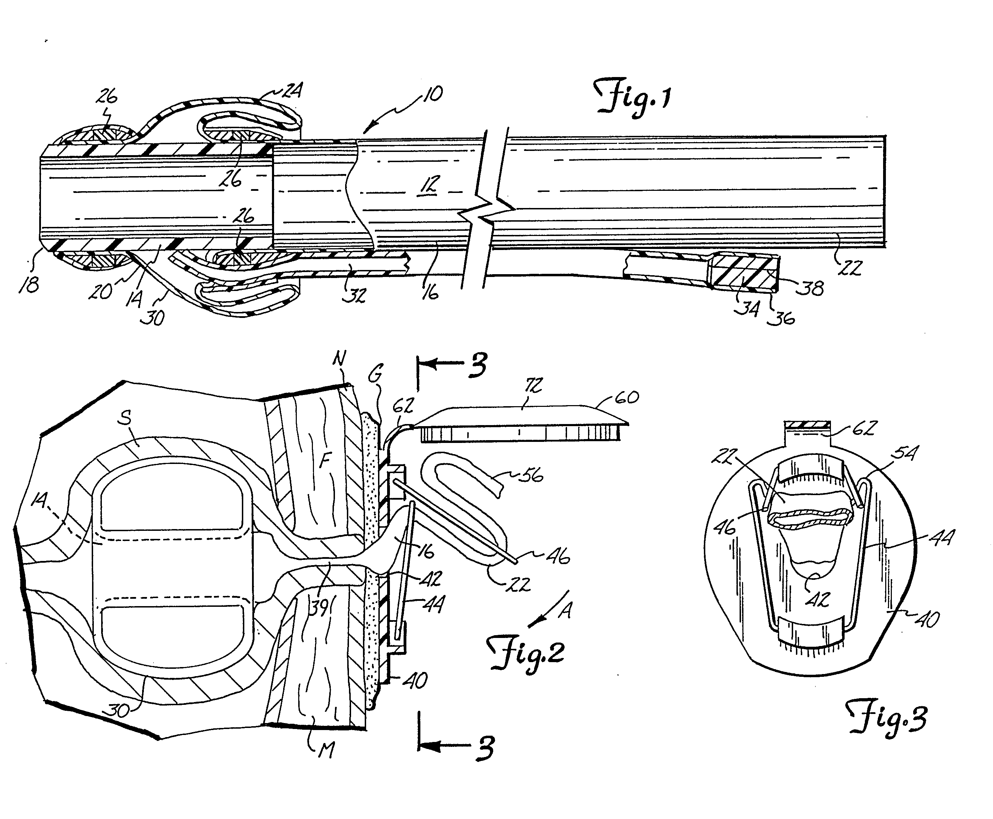

The device of the invention is shown in Figure 1 as (10) and comprises a drainage tube (12) having an inner end portion (14) and an outer end portion (16). The walls of the inner end portion (14), although desirably soft and having some flexibility, are nonetheless sufficiently rigid as to maintain a generally circular, open cross-section within the stoma of a patient. The inner end portion of the drainage tube desirably is made of silicone rubber or similar material, and may, for example, have an outer diameter of about 13 mm and a wall thickness of about 2 mm. The inner end (18) is desirably beveled or rounded to avoid tissue irritation.

The outer end portion (16) is provided with a proximal end (20) which is connected to the inner end portion (14) of the drainage tubing, and a distal end
portion (22) which, in normal usage, protrudes forwardly, that is, distally, from the stoma of a patient. The outer end portion (16) desirably also is
made of silicone rubber and has approximately the same
external diameter as does the inner end portion (14).
Whereas the inner end portion (14) is relatively rigid, however, the outer end portion (16) is limp and
readily collapsed by the radially inwardly directed
forces generated by the bowel. As will be described
in greater detail below, the limpness of the walls of
the outer end portion (16) permit this portion to
yield and collapse as it passes through the stoma, and
this in turn appears to reduce the production of mucus
in the stoma.

In the embodiment shown in Figure 1, the proximal end (20) of the outer drainage tube portion is
drawn snuggly over the more rigid inner end portion
(14). It will be understood that the walls of the
outer end portion (16) of this embodiment are quite
elastic, and the limp tubing is folded forwardly upon
itself as shown at (24) in Figure 1 to provide an excess of material at this location. Relatively rigid
bands (26) encircle and tightly bind the outer end
portion to the inner end portion near ends of the
latter, and, further, outer bands (28) of, e.g., silicone rubber may be employed to cover the rigid bands
(26). The rigid bands are spaced along the length of
the inner end portion (14) of drainage tubing to define, with the folded portion of the outer end portion, an inflatable balloon structure (30). An inflation tube (32) extends from within the inflatable
balloon structure (30) forwardly along the length of
the outer end portion, and terminates in a fluid retention valve through which fluid (e.g., water) under pressure may be injected into the balloon structure and which prevents the escape of such fluid. In the embodiment depicted, the fluid retention valve comprises a plug (34) of silicone rubber and an end tube (36) that radially compresses the plug and that connects the plug to the end of the inflation tube (32). The plug may be provided with a preformed hole pierced through its length, as designated at (38) such that a blunt-nosed hypodermic needle may be forced through the pierced hole in the plug to communicate with the inflation tube, the plug sealing behind the blunt-nosed needle as it is withdrawn from the plug. The inflation tube (32) may be adhered to the walls of the limp outer portion of drainage tubing if desired to restrain the inflation tube from becoming tangled or otherwise out of position.

Referring now to Figure 2, which shows an ostomy device of the invention in position within the stoma of a patient, the stoma is designated generally as "S", the facia as "F", the fat and muscular layer generally as "M" and the abdominal skin as "N". The device of the invention has been inserted in the stoma "S" so that the inner end portion (14) is deep to (that is, proximal of) the facia "F". The balloon structure (30) is inflated with saline solution by injecting the same into the balloon structure through the inflation tube (32), and the limp, outer end portion of the drainage tubing (16) is then pulled outwardly, that is, distally, by the patient to cause the portion of the stoma that is distended through inflation of the balloon structure (30) to bear forwardly against the facia "F", the facia thus serving to positively retain within the stoma the balloon structure (30) and hence the inner end portion of the drainage tube. As noted in Figure 2, the limp walls of the outer end portion are easily collapsed, as shown at (39), by the walls of the bowel. When the bowel contents are to be eliminated, the walls of the stoma surrounding the collapsed portion (36) will distend or dilate in the normal fashion to permit the passage of bowel contents through the outer end portion (16).

The distal end (22) of the outer portion of the drainage tube should not only be maintained in tension to maintain the proper seating of the distended portion of the stoma against the facia, but also must be capable of being sealed off with a waterproof seal to avoid the unintentional escape of bowel contents. This can be accomplished, if desired, through the use of a device such as that depicted in U.S. Patent 4,381,765. More desirably, however, an improved tensioning and sealing device is employed such as that depicted in Figures 2-7, all of which will now be disclosed in greater detail.

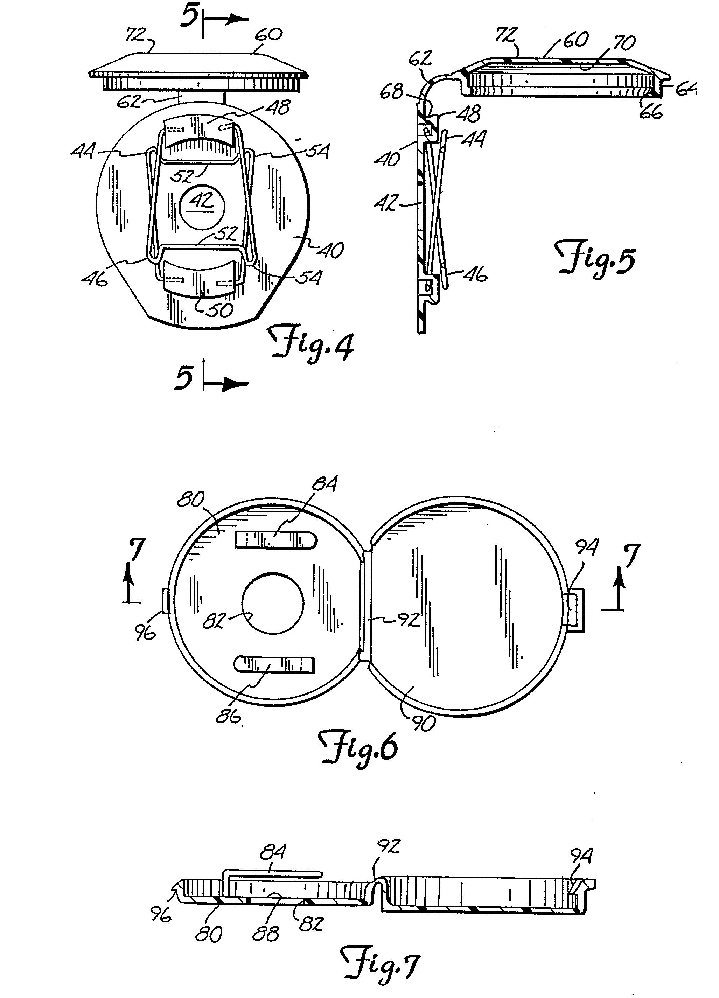

Referring now to Figures 4 and 5, a generally circular substantially rigid plate is shown as (40), the plate having a central aperture (42) through which may extend the externally protruding portion of a drainage tube such as that shown at (16) in Figures 1 and 2. A pair of generally "U"-shaped wire bails (44), (46) have their free ends rotatably mounted respectively in mounting blocks (48), (50), the mounting blocks being spaced on opposite sides of the aperture (42) and the mounting blocks and bails being so positioned and spaced as to permit the bails to be folded one upon another as shown in Figure 4. Each bail, it will be noted, is provided with a broad central portion (52) which is desirably straight and which further is preferably substantially wider - preferably at least about 1.2 times wider – than the diameter of the aperture (42). Further, the wire bails preferably have small outwardly extending portions (54) at either end of the central portion to restrain sideways movement of tubing bent about the central portion (52). A generally circular cover (60) is hingedly mounted at one side to a side of the plate (40) so that the cover may be pivoted downwardly upon the plate. Preferably, the cover and plate are molded as a single unit, and the hinge connection in this case takes the form of a flexible, thin-walled strap (62). Means are provided to releasably hold the cover upon the plate. As shown in the embodiment of Figures 2-5, the rim (64) of the generally dish-shaped cover has an inwardly protruding lip (66) which is oriented to come into contact with and slip or snap into the small grooves (68) formed on the outer circumferential surface of the bail mounting blocks (48), (50).

Referring particularly to Figure 2, the plate (40) is placed against the skin "N" of a patient, a thin layer of gauze "G" being placed between the plate and skin as desired or needed. The proximal end (22) of a protruding drainage tube such as that shown at (16) is inserted forwardly through the aperture (42) in the plate, and by simultaneously pulling the tubing outwardly and pressing the plate against the skin of the patient, the skin, fat and muscle layer "M" and facia are, in effect, pinched between the distended bowel portion and the plate, thereby securely anchoring the drainage tubing against movement. The drainage tubing may then be bent upwardly and around the central portion (52) of bail (44), as shown in Figure 3, the bail (44) then being pivoted to lie flatly adjacently the outer face of the plate (40). The tubing length is then bent about the central portion (52) of the opposing bail (46), as shown in Figure 2, and that bail is then similarly pivoted downwardly as shown by the arrow "A" in Figure 2 to lie flatly adjacent the outer face of the plate (40). In this manner, the tubing is provided with a folded, flattened, generally "Z"-shaped orientation. The terminal end portion (56) (Figure 2) may be trimmed, or may be merely folded atop the Z-shaped bent portion of the tubing. When the cover (60) is snapped onto the plate (40), the inner surface (70) bears inwardly against the folded-over portion of tubing, holding the same in place and preventing it from unraveling or creeping under tension. The outer surface (72) of the cover (60) may be completely smooth and skin-toned, or may be decorated or provided with various designs or coverings as desired. The cover not only presses inwardly upon and immobilizes the Z-shaped tubing carried by the plate (40), but additionally shields the tubing from view and presents a smooth, not unpleasing design to an observer.

It has been found that when the outer end portion of the drainage tube is of limp, collapsible material such as that described above in connection with Figures 1 and 2, the folding of the drainage tube in the manner depicted in Figure 2 provides an extremely satisfactory waterproof seal and further tends to completely prevent the tubing from "creeping" about the bails when maintained under continuous tension. Thus, when the drainage tubing device shown in Figures 1 and 2 is employed with the plate and cover depicted in Figures 2-5, the prosthesis is held firmly and reliably in place.

Figures 6 and 7 depict a modified form of plate and cover. The plate, designated (80), is generally circular and is provided with a central aperture (82) similar to, and having the same purpose as, the aperture (42) in the embodiment depicted in Figure 4. Arising on opposite sides of the aperture are a pair of oppositely extending parallel fingers (84), (86) spaced from but generally parallel to the outer surface (88) of the plate. The cover (90) is hinged at one edge to an edge of the plate by means of a thin-walled hinge (92), and the cover is provided with a latch hook (94) capable of hooking over and latching onto an outwardly extending shoulder (96) formed on the mating portion of the plate, all as shown in Figure 7. In use, the protruding section of drainage tube passes distally through the aperture (82) and then is led first beneath and is bent around the finger (84), thence is doubled back and led beneath and bent around the finger (86), and thence extends again toward the finger (84), thereby providing the tubing with a generally "Z"-shaped configuration. The inner surface (96) of the cover is so oriented that when the cover is snapped downwardly upon the plate (80), the inner surface (96) of the cover comes into contact with and presses inwardly against the folded over portions of tubing, thereby anchoring the same in place.

Various changes can be made to the prostheses of the present invention. For example, although the limp, collapsible section of drainage tubing in the embodiment shown in Figures 1 and 2 forms an integral part of the balloon structure shown as (30), this section of tubing need not be elastic and may instead merely be cemented at its rearward end to the more
rigid tubing (14), a separate length of tubing being
employed to form the balloon structure (30).

0169967

- 12 -

WHAT IS CLAIMED IS:

1.	An ostomy prosthesis connectable to the stoma of a patient for controlling continence and characterized by including a drainage tube having inner and outer end portions, the inner end portion being receivable in the stoma of a patient deep to the facia and having walls of sufficient rigidity as to maintain a generally circular, open cross-section within the stoma of a patient, the outer end portion being connected to and communicating with the inner end portion and having limp walls that are readily collapsible by the stoma of a patient; reversible securing means carried by the inner end portion for reversibly securing the same within a stoma deep to the facia, and sealing means carried by the outer end portion for releasably sealing the latter against escape of bowel contents.

2.	The prosthesis of Claim 1 further characterized in that the inner and outer end portions have adjacent ends, and including means coaxially and sealingly joining said ends.

3.	The ostomy prosthesis of Claim 1 further characterized in that said securing means comprises inflatable balloon means carried concentrically of the inner end portion and being substantially coextensive with the forward end of said inner end portion.

4.	The ostomy prosthesis of Claim 1 further characterized in that said outer end portion is of elastic material and extends coaxially over said inner end portion, means concentrically sealing the outer end portion to the inner end portion at at least two points spaced along the length of the inner end portion, the latter defining, with the overlying outer end portion, inflatable balloon means, the device including means for inflating said balloon means.

5.      The ostomy prosthesis of Claim 1 further characterized in that said means for releasably sealing the outer end portion of the drainage tube comprises means carried by the tube exterior of the patient for releasably shaping the limp, readily collapsible outer end portion in a bent, collapsed, generally Z-shaped orientation to seal said portion against the escape of bowel contents, said sealing means including cover means overlying said bent, collapsed portion to maintain the same in the bent, sealed orientation.

6.      An ostomy prosthesis connectable to the stoma of a patient for controlling continence and characterized by including a drainage tube having inner and outer end portions, the inner end portion being insertable in the stoma of a patient and having walls of sufficient rigidity as to retain a generally circular, open cross-section within the stoma, securing means carried by the inner end portion for securing it to the stoma deep to the facia, and sealing means for releasably sealing the outer end portion of the drainage tube, said sealing means comprising plate means having an aperture through which the outer end of the drainage tube passes, the plate means being adjustable along the length of the outer end portion of the drainage tube to press toward and against the patient's skin, the plate means including means about which the outer end portion of the drainage tube may be bent in a Z-shaped orientation to seal the same, and removable cover means carried by the plate means for covering the shaped outer end portion of the drainage tube and for compressing the

Z-shaped tubing lengths so that the same lie generally parallel to one another against and adjacent the plate, the cover means and plate means together forming a compartment within which is compressed the bent outer end portion of the drainage tube to prevent the same from escaping from the compartment.

7.       The ileostomy prosthesis of Claim 6 further characterized in that said means about which the outer end portion of the drainage tube is bent comprises first and second bail means pivotally connected to the plate means on opposite sides of the aperture therethrough, the bail means being foldable toward one another across the axis of the aperture and against the outer end portion of the drainage tube to shape the latter in said Z-shaped configuration.

8.       The ostomy prosthesis of Claim 6 further characterized in that the cover means comprises a generally dish-shaped cover hingedly attached along one edge thereof to an edge of the plate means and forming with the plate means a substantially complete enclosure for the folded section of the drainage tube, the cover means having an internal pressural surface so oriented with respect to the plate means as to press against the folded portion of the drainage tube when the cover means is pivoted closed upon the plate means to thereby maintain in place the folded section of the drainage tube within the enclosure.

Fig.1

Fig.2

Fig.3

Fig.4

Fig.5

Fig.6

Fig.7

## European Patent Office

## EUROPEAN SEARCH REPORT

Application number

EP 85 30 4254

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | US-A-4 381 765 (BURTON) <br> * claim 1; figures * | 1 | A 61 F 2/48 <br> A 61 F 5/44 |
| Y | GB-A-1 492 946 (RONNQUIST) <br> * page 1, lines 23-32; figure * | 1 | |
| A | US-A-3 802 418 (CLAYTON) | | |
| D,A | US-A-4 119 100 (RICKETT) | | |
| D,A | US-A-3 828 782 (POLIN) | | |
| | ----- | | TECHNICAL FIELDS SEARCHED (Int. Cl.4) |
| | | | A 61 F 2/00 <br> A 61 F 5/00 <br> A 61 M 25/00 |

The present search report has been drawn up for all claims

| Place of search | Date of completion of the search | Examiner |
|---|---|---|
| BERLIN | 06-09-1985 | KANAL P K |